# DEMANDE DE BREVET EUROPEEN

(11) **EP 2 602 788 A2**
(43) Date de publication de la demande: **12.06.2013**
(21) Numéro de dépôt: 12196004.1
(22) Date de dépôt: 07.12.2012
(51) Int. Cl.: G10K 11/00, A61N 7/02, A61B 8/00

(54) **Transducteur ultrasonore à élément actif supporté**

(30) Priorité: 07.12.2011 FR 1161291
(71) Demandeur: Imasonic, 70190 Voray sur L'Ognon (FR)
(72) Inventeur: Fleury, Gérard, 251125 POUILLEY LES VIGNES (FR); Chupin, Laurent, 25620 MAMIROLLE (FR); Berriet, Rémi, 25000 BESANCON (FR); Guey, Jean-Luc, 25480 ECOLE-VALENTIN (FR)
(74) Mandataire: Regimbeau

(57) **Abrégé**

L'invention concerne un transducteur (1) ultrasonore comportant :
- un boîtier (2),
- un élément actif (3) pour générer et/ou recevoir des ondes ultrasonores, et
- un élément support (4) situé à l'arrière de l'élément actif (3), caractérisé en ce que l'élément support (4) comporte une face avant complémentaire d'une face arrière de l'élément actif (3), ledit élément support (4) étant conformé de sorte à ce que la face arrière complémentaire de l'élément actif (3) soit en appui simple sans couplage acoustique significatif avec la face avant dudit élément support (4), l'élément actif (3) et l'élément support (4) étant couplés thermiquement, et dans lequel la face avant de l'élément support (4) et la face arrière de l'élément actif (3) sont conformées pour définir entre elles, lorsque l'élément support (4) et l'élément actif (3) sont en appui simple, une couche discontinue de gaz (5) d'une épaisseur moyenne comprise entre 0,5 µm et 50 µm suffisamment faible pour favoriser un couplage thermique entre l'élément support (4) et l'élément actif (3).

## Description

La présente invention concerne un transducteur ultrasonore, pour émettre et/ou recevoir un faisceau d'ondes ultrasonores. Pour certaines applications biologiques, les ultrasons sont générés avec une forte intensité acoustique, par exemple pour la destruction de tissus cancéreux, la dislocation de caillots sanguins ou de calculs, ou encore la libération de substances chimiques. D'autres applications se placent entre autres dans le domaine industriel, par exemple en sonochimie, dans le domaine de la communication et du transfert d'énergie, dans le domaine sous-marin, dans le domaine pétrolier. Lorsque les ultrasons de puissance sont focalisés, on utilise communément l'acronyme HIFU pour l'anglais "High Intensity Focused Ultrasound".

Ces ultrasons de forte intensité engendrent des effets physiques ou biophysiques dans les milieux dans lesquels ils sont générés : l'effet peut être thermique ou mécanique et, dans le cadre d'applications biologiques, peut être biophysique en contribuant, par exemple, à l'activation de substances chimiques actives, au transfert de gènes ou au contrôle de perméabilité d'une membrane. Lorsque le faisceau d'ultrasons est focalisé, l'effet est localisé au voisinage du point focal.

La génération d'ultrasons de forte densité de puissance moyenne par le transducteur cause l'échauffement de celui-ci, dégradant ses performances. De plus, l'échauffement du transducteur peut entraîner sa déformation par dilatation, ce qui peut se traduire par un déplacement du point focal où convergent les faisceaux d'ultrasons dû au changement de géométrie du transducteur. L'échauffement des transducteurs de l'état de la technique et leur manque de rigidité impose la limitation à de faibles niveaux de leur puissance moyenne d'émission par unité de surface. En outre, les transducteurs de l'état de la technique ne donnent pas entière satisfaction, dans la mesure où ceux-ci sont habituellement fragiles.

### PRESENTATION GENERALE DE L'INVENTION

L'invention propose de pallier au moins un de ces inconvénients, préférentiellement tous. A cet effet, on propose un transducteur ultrasonore, notamment de thérapie, comportant :
- un boîtier,
- un élément actif pour générer et /ou recevoir des ondes ultrasonores,
   et
- un élément support situé à l'arrière de l'élément actif,
l'élément support comportant une face avant complémentaire d'une face arrière de l'élément actif, ledit élément support étant conformé de sorte à ce que la face arrière complémentaire de l'élément actif soit en appui simple sans couplage acoustique significatif avec la face avant dudit élément support, l'élément actif et l'élément support étant couplés thermiquement, et dans lequel la face avant de l'élément support et la face arrière de l'élément actif sont conformées pour définir entre elles, lorsque l'élément support et l'élément actif sont en appui simple, une couche discontinue de gaz d'une épaisseur moyenne comprise entre 0,5 µm et 50 µm suffisamment faible pour favoriser un couplage thermique entre l'élément support et l'élément actif.

L'invention est avantageusement complétée par les caractéristiques suivantes, prises seules ou en une quelconque de leur combinaison techniquement possible:
- la couche discontinue de gaz correspond au volume de gaz résiduel créé par des rugosités de la face avant de l'élément support et de la face arrière de l'élément actif lorsque l'élément support et l'élément actif sont en appui simple;
- le transducteur comporte en outre des organes de maintien en dépression adaptés pour maintenir la couche discontinue de gaz à une pression inférieure d'au moins 200 mbar à la pression extérieure à laquelle est soumise la face avant de l'élément actif 3;
- l'écart moyen arithmétique des rugosités par rapport à la ligne moyenne d'au moins une face parmi la face arrière de l'élément actif et la face avant de l'élément support est compris entre 0,1 µm et 20 µm;
- l'élément actif est encastré dans le boîtier par l'intermédiaire d'au moins un joint élastique, et un effort mécanique de poussée de l'élément support par rapport au boîtier est généré au moyen d'éléments de plaquage afin de maintenir l'élément actif en appui sur l'élément support par une réaction dudit joint élastique à l'effort mécanique;
- l'élément actif comporte des zones de souplesse;
- le transducteur est équipé d'un système de refroidissement mettant en oeuvre la circulation d'un fluide caloporteur;
- l'élément actif comporte une pluralité d'électrodes sur sa face arrière, et l'élément support comporte une pluralité d'orifices traversant en regard d'au moins une partie de ladite pluralité d'électrodes;
- l'élément support est composé de matériaux amagnétiques compatibles avec une utilisation en imagerie par résonnance magnétique;
- l'élément support est composé d'une pluralité de sous-éléments support mécaniquement reliés au boîtier, chacun desdits sous-éléments support étant au contact de l'élément actif.

L'invention concerne également un procédé d'utilisation d'un transducteur selon l'invention, comportant :
- une émission d'un faisceau d'ondes ultrasonores par l'élément actif, et
- un support de l'élément actif par un élément support sans couplage acoustique significatif.

### PRESENTATION DES FIGURES

D'autres caractéristiques et avantages de l'invention ressortiront encore de la description qui suit, laquelle est purement illustrative et non limitative. Cette description doit être lue sur la base des dessins annexés, sur lesquels :
- la figure 1 représente une vue en coupe d'un mode de réalisation possible de l'invention ;
- les figures 2 et 3 illustrent différentes variantes possibles de l'invention ;
- les figures 4a à 4e présentent différentes configurations d'élément actif présentant des zones de souplesse ;
- la figure 5 est une courbe illustrant l'évolution de la conduction thermique de l'air en fonction de la pression ;
- la figure 6 est une vue de dessus d'un élément support;
- les figures 7a à 7c sont des vues en coupe selon AA d'un élément support de la figure 6;
- les figures 8 et 9 sont des vues de dessus d'un élément support recouvrant une partie d'un élément actif ;
- la figure 10 représente une vue en coupe agrandie de l'interface entre l'élément actif et l'élément support;
- les figures 11a, 11b et 11c représentent respectivement une vue de côté, de dessus et en coupe d'un transducteur selon un mode de réalisation possible de l'invention, dans laquelle l'élément support est constitué d'une pluralité de sous-éléments.

### DESCRIPTION DETAILLEE DE L'INVENTION

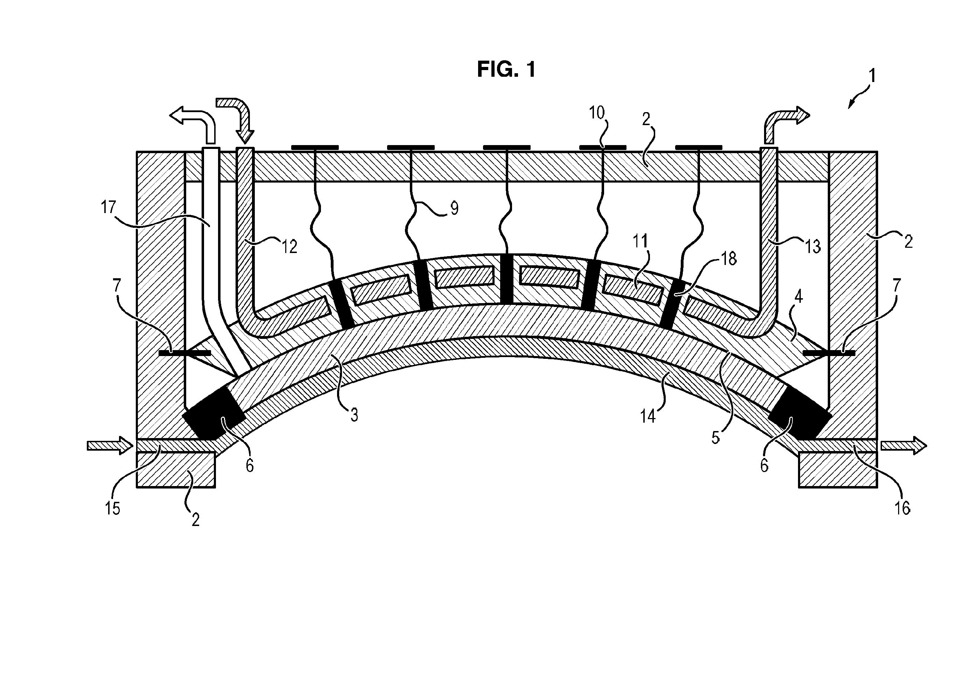

En référence à la figure 1, le transducteur 1 selon l'invention comporte un boîtier 2, un élément actif 3 pour générer et/ou recevoir des ondes ultrasonores, et un élément support 4 situé à l'arrière de l'élément actif 3. L'élément support 4 peut faire partie du boîtier ou en être distinct.

L'élément actif 3 est généralement constitué principalement d'un matériau piézoélectrique, éventuellement piézocomposite, éventuellement multicouche, et d'un ensemble d'au moins deux électrodes qui permettent de créer un champ électrique dans l'épaisseur du matériau piézoélectrique. De préférence, une ou plusieurs couches d'adaptation acoustique sont intégrées dans cet élément actif, sur la face avant de l'élément actif 3, pour faciliter le transfert acoustique vers l'avant du transducteur 1.

La description qui suit sera faite non limitativement à des fins d'illustration en référence à un tel élément actif 3 mettant en oeuvre des phénomènes piézoélectriques. Cependant, l'élément actif 3 peut être tout dispositif électroacoustique tel qu'un transducteur capacitif, par exemple un transducteur capacitif micro-usiné (ou CMUT pour l'anglais Capacitive Micromachined Ultrasonic Transducer), un transducteur électrostrictif, ...

L'élément support 4 comporte une face avant complémentaire d'une face arrière de l'élément actif 3, ledit élément support 4 étant conformé de sorte que la face arrière complémentaire de l'élément actif 3 soit en appui simple, sans collage ni mouillage, de manière à minimiser le couplage acoustique avec la face avant dudit élément support 4 de sorte que l'élément actif 3 et l'élément support soient en appui simple sans couplage acoustique significatif.

Le couplage acoustique entre l'élément actif 3 et l'élément support 4 est jugé significatif si plus de 10 % de l'énergie acoustique produite par l'élément actif 3 est transmise à l'élément support 4. L'énergie transmise à l'élément support 4 peut être estimée par comparaison entre l'énergie fournie à l'élément actif 3 et l'énergie acoustique recueillie à l'avant de l'élément actif 3, avec et sans l'élément support 4, en prenant soin de prendre en compte des facteurs additionnels tels que l'énergie thermique ou la dispersion acoustique dans l'air.

La face avant de l'élément support 4 et la face arrière de l'élément actif 3 sont en appui simple au moyen de zones de contact au niveau desquelles la face avant de l'élément support 4 et la face arrière de l'élément actif 3 sont en contact mécanique.

La face avant de l'élément support 4 présente une forme sensiblement similaire à celle de la face arrière de l'élément actif 3, de sorte que ces deux faces s'ajustent entre elles lorsque l'élément actif 3 et l'élément support 4 sont en appui simple afin que lesdites zones de contact soient réparties, de préférence uniformément, sur la surface de la face avant de l'élément support 4 et de la face arrière de l'élément actif 3.

Cependant, lesdites zones de contact représentent une surface totale bien inférieure à la surface de la face avant de l'élément support 4 et de la face arrière de l'élément actif 3, dans la mesure où seules des zones en saillie de la face avant de l'élément support 4 et de la face arrière de l'élément actif 3 se touchent pour réaliser l'appui simple. Ainsi, une majorité de la surface de la face avant de l'élément support 4 n'est pas en contact avec la surface de la face arrière de l'élément actif 3, et vice-versa.

L'élément support 4 sert de référence de forme et de renfort mécanique, permettant notamment au transducteur 1 de résister aux chocs. En outre, en cas de dilatation thermique de l'élément actif 3, en raison de son utilisation ou des conditions extérieures, l'élément support 4 maintient l'élément actif dans une forme utile. L'élément support 4 présente une rigidité supérieure à l'élément actif 3 afin de constituer une référence de forme pour celui-ci.

En outre, pour permettre la dilatation de l'élément actif 3 en appui simple sur l'élément support 4, au moins un joint élastique souple 6 est disposé à l'interface entre l'élément actif 3 et le boîtier 2. Suivant les configurations, le joint élastique 6 peut être en périphérie de l'élément actif 3 ou sur le bord d'un perçage central traversant l'épaisseur de l'élément actif 3. Le joint élastique 6 assure également la fixation de l'élément actif 3 sur le boîtier 2 et l'étanchéité de l'espace défini par le boîtier 2 et l'élément actif 3, notamment par rapport à l'avant de l'élément actif 3 susceptible de se trouver en contact avec de l'eau.

Ce joint élastique souple 6 présente une rigidité mécanique inférieure à celle de l'élément actif 3 et par conséquent absorbe toute dilatation ou déformation dudit élément actif 3, sans perte d'étanchéité, en même temps qu'il le plaque contre l'élément support 4. Le ou les joints élastiques 6 peuvent par exemple être en élastomère silicone.

Dans le cas de transducteurs focalisés, et ainsi que représenté en coupe sur la figure 1, l'élément support 4 et l'élément actif 3 présentent une forme globalement concave, par exemple une forme de calotte sphérique, définissant un point focal vers lequel convergent les faisceaux d'ondes sonores émis par l'élément actif 3.

En cas de déformation, suite à un choc ou par dilatation thermique, de l'élément actif 3, celui-ci peut prendre une forme qui ne définirait plus de point focal ou qui définirait une distribution de pression acoustique différente dans le champ. Dans la mesure où l'utilisation d'un tel transducteur 1 est justement liée à la concentration de puissance acoustique à un point focal précis, un transducteur 1 présentant un élément actif 3 déformé peut présenter des inconvénients voire des risques dans son utilisation.

Cependant, l'élément support 4 doit rester acoustiquement découplé de l'élément actif 3 pour ne pas modifier le fonctionnement de l'élément actif 3 lorsque celui-ci émet des ondes ultrasonores. En effet, un couplage acoustique réduirait, par la transmission d'énergie acoustique vers l'élément support 4, le rendement du transducteur.

En outre, l'élément actif 3 et l'élément support 4 sont couplés thermiquement, sans couplage acoustique significatif. Ce couplage thermique permet de drainer la chaleur émise au cours des cycles d'émission d'ondes ultrasonores par l'élément actif 3 depuis celui-ci vers l'élément support 4, autorisant ainsi une puissance accrue d'émission ultrasonore et/ou un fonctionnement prolongé.

Le couplage thermique entre l'élément actif 3 et l'élément support 4 est jugé satisfaisant si la résistance thermique de l'interface entre l'élément actif 3 et l'élément support 4 est inférieure à 0,002 m².K.W⁻¹. De préférence, la résistance thermique est inférieure à 0,0008 m².K.W⁻¹. Une valeur de résistance thermique de 0,002 m².K.W⁻¹ correspond à une épaisseur d'air de 50 µm, tandis que celle de 0,0008 m².K.W⁻¹ correspond à une épaisseur d'air de 20 µm. Ces valeurs sont déterminées à une pression de 1 bar.

A cet effet, la face avant de l'élément support 4 et la face arrière de l'élément actif 3 sont conformées pour définir entre elles une couche discontinue de gaz 5 d'une épaisseur moyenne comprise entre 1 µm et 50 µm. L'épaisseur moyenne de cette couche doit être suffisamment faible pour favoriser le transfert de chaleur entre l'élément actif 3 et l'élément support 4, afin d'assurer le maintien d'un couplage thermique entre l'élément actif 3 et l'élément support 4.

Le gaz préférentiellement utilisé en raison de sa disponibilité et de son faible coût est l'air, et la description qui suit sera faite en référence à l'air en tant que gaz constituant la couche discontinue. Néanmoins, d'autres gaz pourraient être utilisés, par exemple dans la mesure où ceux-ci permettent de réaliser un meilleur couplage thermique tout en minimisant le couplage acoustique ou minimisent l'oxydation des surfaces, tels que l'azote, l'hélium, le dioxyde de carbone, ....

L'épaisseur de la couche de gaz 5 dépend notamment de la fréquence des ondes ultrasonores émises par le transducteur 1. Pour des fréquences de l'ordre de 1MHz, typique de l'utilisation de tels transducteurs, une épaisseur moyenne de couche d'air 5 supérieure à 50 µm ne permet pas d'obtenir un transfert thermique satisfaisant, tandis qu'une épaisseur inférieure à 1 µm entraîne un risque de transmission d'énergie acoustique vers l'élément support 4. Ainsi, l'épaisseur moyenne de la couche d'air 5 est préférentiellement comprise entre 1 µm et 20 µm, afin d'assurer un découplage acoustique entre l'élément actif 3 et l'élément support 4, tout en permettant un transfert thermique satisfaisant depuis l'élément actif vers l'élément support 4 à travers cette couche d'air 5.

L'épaisseur de la couche de gaz 5 entre l'élément actif 3 et l'élément support 4 peut notamment résulter de tolérances de forme entre l'élément actif 3 et l'élément support 4, et notamment entre la face arrière de l'élément actif 3 et la face avant de l'élément support 4, afin de définir une épaisseur minimum de la couche de gaz 5 lorsque l'élément actif 3 est en appui simple sur l'élément support 4.Cependant, des tolérances de l'ordre du micron sont difficiles à respecter sur des pièces de grand diamètre sous forme de calotte sphérique, ce qui peut se traduire par des inhomogénéités dans l'épaisseur de la couche de gaz.

Or l'épaisseur d'air doit être aussi homogène que possible à l'échelle de la surface totale de l'interface entre la face arrière de l'élément actif 3 et la face avant de l'élément support 4, de manière à ne pas créer d'inhomogénéité sur la surface, par exemple en créant des zones mieux refroidies que d'autres.

Par ailleurs, en fonctionnement, le transducteur 1 s'échauffe et l'élément actif 3 se dilate et donc l'épaisseur définie par les seules tolérances de forme change également. L'épaisseur locale de la couche de gaz 5 évolue donc en cours d'utilisation.

Par conséquent, dans un mode de réalisation préférentiel, l'épaisseur de la couche 5 de gaz entre l'élément actif 3 et l'élément support 4 résulte notamment des états de surface des deux éléments en contact. Ainsi, la couche de gaz 5 peut correspondre au volume d'air résiduel créé par les rugosités de la face avant de l'élément support 4 et de la face arrière de l'élément actif 3, ou seulement par la rugosité de l'une des deux surfaces, lorsque l'élément actif 3 est en appui simple sur l'élément support 4

La rugosité étant sensiblement la même sur toute la superficie des faces en contact, l'homogénéité de la qualité du contact est garantie sur toute cette superficie. L'état de surface résulte notamment du choix des procédés de fabrication et de la nature des matériaux en jeu, et plus spécialement du traitement de surface réalisé sur lesdits matériaux. Des stries peuvent en outre être réalisées mécaniquement sur ces surfaces afin de définir des paramètres relativement précis de rugosité.

La rugosité définit des saillies et des creux. La face arrière de l'élément actif 3 et la face avant de l'élément support 4 sont donc en appui simple par contact mécanique au niveau de certaines saillies desdites faces, tandis que les creux et les autres saillies définissent alors les volumes de gaz qui constituent la couche discontinue de gaz 5.

De préférence, l'écart moyen arithmétique Rₐ des rugosités par rapport à la ligne moyenne d'au moins une face parmi la face arrière de l'élément actif 3 et la face avant de l'élément support 4 est compris entre 0,1 µm et 20 µm, et de préférence entre 0,1 µm et 10 µm.

Ainsi, par exemple, la rugosité de la face arrière de l'élément actif 3 présente un écart moyen arithmétique Rₐ par rapport à la ligne moyenne de la face arrière compris entre 1 µm et 10 µm, de préférence entre 2 µm et 8 µm avec une amplitude moyenne de la rugosité comprise entre 10 et 20 µm.

De même, par exemple, la rugosité de la face avant de l'élément support 4 présente un écart moyen arithmétique Rₐ par rapport à la ligne moyenne de la face avant compris entre 0,2 µm et 5 µm, de préférence entre 0,5 µm et 2 µm avec une amplitude moyenne de la rugosité d'environ 5 µm.

Les rugosités décrites ici sont issues de mesures réalisées grâce à un rugosimètre mécanique, avec une pointe en contact avec la surface, sur une longueur de mesure de 10 à 20 mm.

De préférence, l'élément actif 3 est principalement composé d'un matériau piézocomposite, préférentiellement des plots de céramique piézoélectrique dans une matrice polymère, telle qu'une résine époxy. L'élément actif 3 est alors composé de deux phases qui réagissent différemment aux procédés de surfaçage; on a ainsi par exemple des "marches" de 10 à 20 µm aux interfaces entre les deux phases de l'élément actif 3.

Le transducteur peut également comprendre plusieurs transducteurs élémentaires au niveau de l'élément actif 3. Ces transducteurs élémentaires doivent restés électriquement isolés entre eux et donc isolés de l'élément support 4. Ainsi, l'élément support 4 peut dans ce cas être constitué de matériaux céramiques ou polymères électriquement isolants et thermiquement conducteurs. Alternativement, un traitement de surface ou un dépôt d'isolant peut être appliqué dans le cas d'un élément support 4 conducteur électrique.

Le matériau constituant l'élément support 4 doit présenter de préférence une rigidité mécanique supérieure à la rigidité de l'élément actif 3 afin de remplir sa fonction de support, ainsi qu'une conductivité thermique élevée. De préférence, le ou au moins l'un des matériau(x) constituant l'élément support 4 présente(nt) une conductivité thermique supérieure à 100 W.m⁻¹.K⁻¹, auquel cas l'épaisseur de l'élément support 4, c'est-à-dire la distance moyenne entre sa face avant et sa face arrière, est typiquement comprise entre environ 10 et 20 mm. Préférentiellement, l'élément support 4 est majoritairement constitué de métal ou de céramique, par exemple en aluminium anodisé ou en alumine.

Il peut également être constitué d'autres matériaux, de préférence répondant aux contraintes imposées pour une utilisation dans une enceinte d'un dispositif d'imagerie par résonnance magnétique (IRM). Ainsi, le matériau constituant l'élément support 4 est de préférence amagnétique tel qu'un métal amagnétique comme du cuivre ou du laiton, ou une céramique par exemple à base de nitrure d'aluminium ou de nitrure de bore, voire encore un polymère chargé spécialement conçu pour des applications thermiques, c'est-à-dire dont la conductivité thermique est au moins de l'ordre de 10 W.m⁻¹.K⁻¹.

Afin d'améliorer la compatibilité IRM, il est avantageux de limiter le volume de métal constituant le transducteur. Une possibilité est alors l'utilisation d'une structure composite métal-résine pour l'élément support 4, dans laquelle les parties métalliques assurent alors le drainage thermique et les parties de résine assurent le renfort mécanique des parties métalliques dont l'épaisseur peut alors être faible.

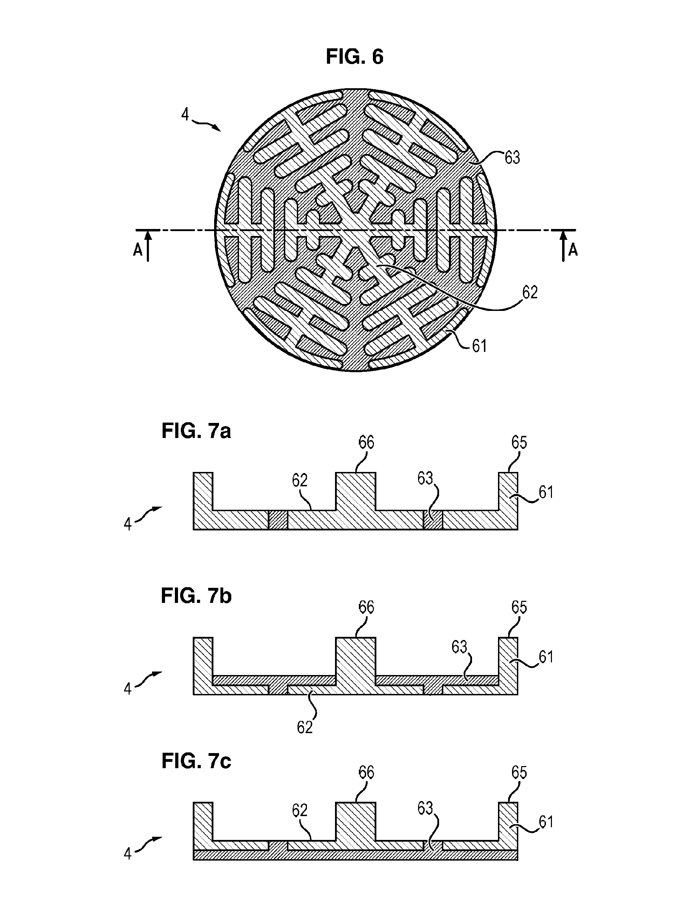

La figure 6 illustre un exemple de structure d'un élément support 4, dans laquelle des parties de résine 63 complètent des parties de métal 61, 62. Ainsi qu'illustré, ces parties métalliques 61, 62 présentent ici une extension radiale afin de permettre de drainer la chaleur produite par l'élément actif 3 vers une surface d'échange avec un élément de refroidissement, actif ou passif.

Ces parties de métal 61, 62 peuvent en outre se décomposer en deux séries se distinguant par leur zone d'échange de chaleur 65, 66 respective. Ces zones d'échange de chaleur sont mises en évidence dans les figures 7a à 7c, qui représentent des vue en coupe selon AA de l'élément support 4 de la figure 6.

Des parties métalliques périphériques 61 présentent ainsi une zone d'échange de chaleur périphérique 65 à la périphérie de l'élément support 4, tandis que des parties métalliques centrales 62 présentent une zone d'échange de chaleur centrale 66 au centre de l'élément support 4.

Ainsi que mis en évidence sur les figures 7, les parties de résine 63 s'étendent de préférence à certains endroits sur toute l'épaisseur de l'élément support 4 . Afin de minimiser la quantité de métal dans l'épaisseur de l'élément support 4, les parties de résine 63 peuvent s'étendre à l'arrière (figure 7b) ou à l'avant (figure 7c) des parties métalliques 61, 62, de sorte que celles-ci soient réduites à des géométries d'épaisseur fine dans l'élément support 4, en s'assurant toutefois que les sections desdites parties métalliques 61, 62 restent suffisantes pour assurer leur rôle de drain thermique.

La résine est choisie pour sa bonne tenue mécanique, notamment pour sa rigidité élevée, supérieure à celle de l'élément actif 3, comme par exemple certaines résines chargées. En effet, la rigidité de l'élément support 4 doit rester supérieure à celle de l'élément actif 3 de manière à constituer une référence de forme pour celui-ci.

Comme représenté sur la figure 1, des fils de reprise 9 et des contacts 18 traversant l'élément support 4 établissent la connexion électrique entre des circuits 10 à l'extérieur ou à l'intérieur du boîtier 2 et des électrodes disposées sur la face arrière de l'élément actif 3, afin de commander l'émission d'ondes ultrasonores par ledit élément actif 3.

Le transducteur 1 représenté sur la figure 1 comporte en outre au moins un système de refroidissement mettant en oeuvre la circulation d'un fluide caloporteur, afin d'évacuer la chaleur en dehors du transducteur 1. Ce système de refroidissement peut être disposé au niveau de l'élément support 4 pour en évacuer la chaleur. Il peut se composer alors de parties creuses 11 ménagées à l'intérieur de l'élément support 4 dans lesquelles circule un fluide caloporteur, par exemple de l'eau.

Une amenée d'entrée 12 sous la forme d'une conduite de fluide traversant le boîtier 2 et pénétrant dans l'élément support 4 permet d'alimenter en fluide caloporteur les parties creuses 11 dudit élément support 4.

Une amenée de sortie 13 sous la forme d'une conduite de fluide traversant le boîtier 2 et pénétrant dans l'élément support 4 permet d'évacuer le fluide caloporteur des parties creuses 11 dudit élément support 4.

De préférence, les parties creuses 11 et les amenées d'entrée 12 et de sortie 13 sont agencées de sorte que le fluide de travail assure un refroidissement homogène de l'élément support 4. Ainsi, plusieurs amenées respectivement d'entrée 12 et de sortie 13 peuvent être régulièrement disposées, de préférence de façon alternée.

Un dispositif extérieur de mise en circulation du fluide caloporteur est en outre alors prévu. Le débit de fluide caloporteur doit être suffisant, par exemple être de 1 litre par minute dans le cas de l'eau, afin d'évacuer une chaleur suffisante pour limiter l'échauffement de l'élément actif 3 au cours de son excitation et pour garantir une homogénéité du refroidissement en conservant une faible différence de température entre le fluide entrant et le fluide sortant.

La présence d'un système de refroidissement mettant en oeuvre la circulation d'un fluide caloporteur au niveau de l'élément support 4 permet l'utilisation de matériaux de plus faible conductivité thermique pour l'élément support 4 par rapport aux exigences précédemment formulées. Dans ce cas, l'épaisseur de l'élément support 4 est plus faible, par exemple inférieure à 5 mm.

Alternativement, ou de préférence de façon additionnelle ainsi qu'illustré par la figure 1, le système de refroidissement peut être disposé au niveau de l'élément actif 3 pour en évacuer la chaleur. Il se compose d'une conduite 14 en surface de l'avant de l'élément actif 3, à l'intérieur de laquelle circule un fluide caloporteur, par exemple de l'eau, définissant une lame d'eau refroidissant l'élément actif 3. Des amenées respectivement d'entrée 15 et de sortie 16 permettent d'alimenter la lame d'eau comme indiqué plus haut dans le cas des parties creuses 11 de l'élément support 4.

D'autres systèmes d'échange thermique peuvent être prévus. Dans le cas où l'élément de support 4 présente une bonne conductivité thermique, le système de refroidissement mettant en oeuvre la circulation d'un fluide caloporteur peut être disposé au niveau du boîtier 2, l'élément support 4 comportant alors une surface d'échange avec le boîtier 2. Le système d'échange thermique peut encore prendre la forme d'ailettes de refroidissement disposées sur l'élément support 4 ou le boîtier 2.

Le transducteur 1 représenté sur la figure 1 comporte des moyens de plaquage pour maintenir le contact en appui simple de l'élément actif 3 et de l'élément support 4.

Ainsi qu'illustré par la figure 1, ces moyens de plaquage peuvent prendre la forme d'organes de maintien en dépression adaptés pour maintenir la couche discontinue de gaz 5 à une pression suffisamment faible par rapport à la pression extérieure à laquelle est soumis l'avant du transducteur. La dépression est d'au moins 200 mbar par rapport à la pression extérieure, et de préférence d'au moins 400 mbar, afin d'assurer le plaquage de l'élément actif 3 sur l'élément support 4.

A titre d'exemple, à une pression atmosphérique normale typiquement d'environ 1 bar soit 10⁵ Pa, la pression est alors de préférence inférieure à 800 mbar, et encore de préférence inférieure à 600 mbar.

Les organes de maintien en dépression illustrés par la figure 1 comprennent une conduite de gaz 17 reliant la couche de gaz 5 à l'extérieur du boîtier, et raccordable à un dispositif d'aspiration pour amener la couche de gaz 5 à la pression voulue. L'étanchéité est assurée entre l'élément support 4 et le boîtier 2 par une solution d'étanchéité, par exemple un joint souple en compression 7, et entre l'élément actif 3 et le boîtier 2 par au moins un joint élastique 6 maintenant l'élément actif 3 encastré dans le boîtier.

La pression réduite de la couche de gaz 5 pourrait également être constante et maintenue uniquement par l'étanchéité apportée par lesdits joints, mais l'aspiration d'air permet de pallier aux défauts éventuels d'étanchéité du joint souple en compression 7, créant en même temps une circulation de gaz au niveau de la couche de gaz 5. L'espace défini par le boîtier 2 à l'arrière de l'élément support peut également être maintenu en dépression, auquel cas le plaquage de l'élément actif 3 contre l'élément support 4 résulte de la force exercée sur la face avant de l'élément actif 3 par la pression extérieure, l'élément support 4 étant solidaire du boîtier 2.

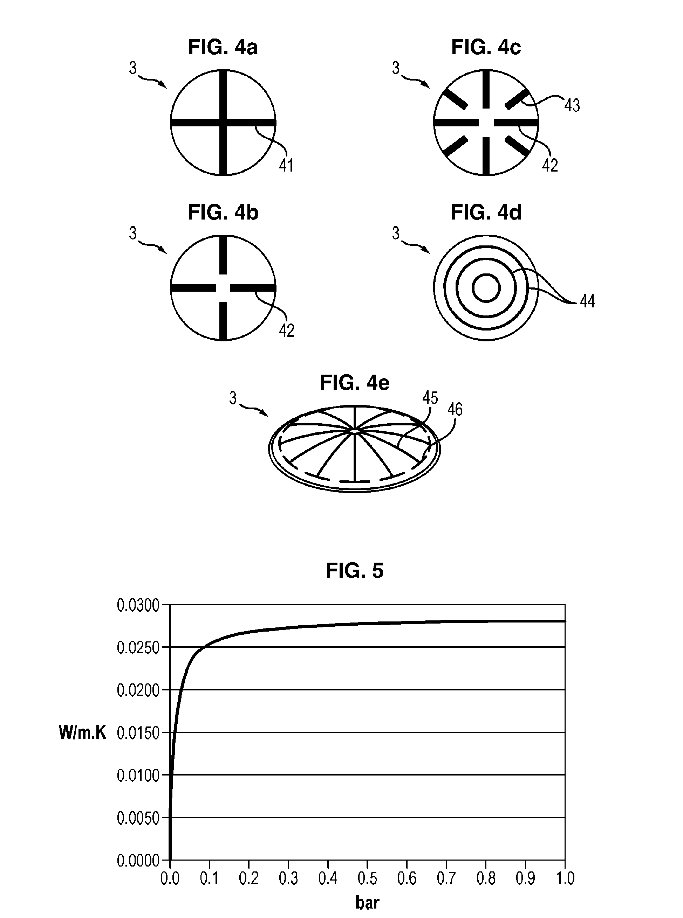

La figure 5 est une courbe illustrant l'évolution de la conduction thermique de l'air, exprimée en W.m⁻¹.K⁻¹, en fonction de la pression, exprimée en bars, notamment pour une couche d'air de 20 µm d'épaisseur à 25°C. On voit que la conductivité thermique reste sensiblement la même pour des pressions comprises entre 200 mbar et 1 bar. En revanche, pour des dépressions plus importantes, notamment inférieures à 100 mbar, la conduction thermique de l'air chute et par conséquent, la qualité du transfert thermique depuis l'élément actif 3 vers l'élément support 4 se dégrade également. La pression absolue devrait donc être supérieure à 100 mbar. De préférence, la pression de la couche de gaz 5 est maintenue au-dessus de 150 mbar, et de préférence encore au-dessus de 200 mbar.

Le gaz contenu dans la couche de gaz 5 peut également avantageusement être en mouvement au moyen d'une aspiration et d'une amenée de gaz. Une circulation de ce gaz permet d'améliorer encore le transfert de chaleur à l'interface entre l'élément actif 3 et l'élément support 4 en évitant l'accumulation de chaleur à cette interface, voire de prévenir des problèmes liés à une présence accidentelle d'humidité à cette interface. En outre, la couche de gaz équipée de la circulation peut constituer un système de refroidissement mettant en oeuvre la circulation du fluide.

De plus, la détente du gaz circulant dans la couche de gaz 5 peut également refroidir avantageusement tant l'élément actif 3 que l'élément support 4.

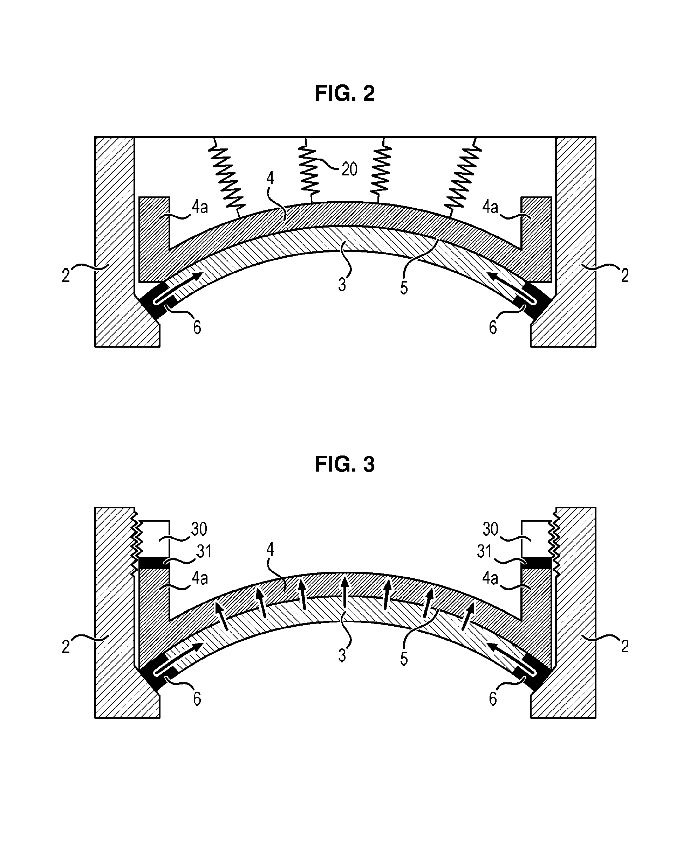

En référence aux figures 2 et 3, l'élément actif 3 est encastré dans le boîtier 2 par l'intermédiaire d'au moins un joint élastique 6, et un effort mécanique de poussée de l'élément support 4 par rapport au boîtier est généré au moyen d'éléments de plaquage 20, 30 afin de maintenir l'élément actif 3 en appui sur l'élément support 4 par une réaction dudit joint élastique 6 à l'effort de poussée. Le joint élastique 6 est alors précontraint.

La direction de réaction des joints 6 élastiques est schématisée par les flèches. Les forces de réaction tangentielles engendrent la déformation de l'élément actif 3 contre l'élément support 4. Le joint élastique 6 présente une rigidité inférieure à celle de l'élément actif 3 et ainsi absorbe les déformations de celui-ci tout en le maintenant sous contrainte par son élasticité.

Ainsi qu'illustré par la figure 2, les éléments de plaquage peuvent être des ressorts 20 en compression reliant le boîtier 2 à l'élément support 4 afin de le maintenir en appui simple contre l'élément actif 3. La direction de réaction des joints 6 élastiques est schématisée par les flèches. On notera que les ressorts peuvent alors également servir de drain thermique pour évacuer la chaleur de l'élément support 4. Le plaquage peut encore être réalisé au moyen de vis de pression.

Dans une autre configuration illustrée par la figure 3, L'élément actif 3 est encastré dans le boîtier 2 par l'intermédiaire d'au moins un joint 6 élastique et les éléments de plaquage peuvent comprendre une bague 30 vissée dans le boîtier de sorte qu'un effort mécanique de poussée pour appuyer l'élément support 4 contre l'élément actif 3 soit transmis audit élément support 4, éventuellement au moyen d'un élément intermédiaire 31 assurant une liaison entre ledit élément de plaquage 30 et l'élément de support 4.

Dans la configuration illustrée par les figures 2 et 3, l'élément support 4 n'est pas fixé au boîtier 2 et comporte un rebord de guidage 4a afin de guider l'élément support 4 dans le boîtier 2. Ce rebord de guidage 4a s'étend de préférence sur toute la périphérie de l'élément support 4, mais peut éventuellement être remplacé par des pattes remplissant la même fonction ou toute autre solution de guidage mécanique.

L'encastrement par un joint 6 souple en périphérie de l'élément actif 3 associé à une précontrainte dudit joint souple 6 par des éléments de plaquage tels que des ressorts ou des éléments vissés permet de se passer éventuellement d'organes de maintien en dépression. En outre, le joint souple 6, ainsi qu'indiqué plus haut, est étanche. Il peut être en élastomère silicone.

Une forme concave de l'élément actif 3 permet en outre d'obtenir un plaquage de l'élément actif 3 contre l'élément support 4 en raison de la dilation thermique de l'élément actif 3 lorsque celui-ci émet des ultrasons et peut donc être suffisant pour maintenir la face arrière de l'élément actif 3 soit en appui simple avec la face avant de l'élément support 4.

Dans la mesure où il est préférable que l'élément actif 3 se déforme pour épouser la forme de l'élément support 4 qui lui sert de référence de forme, l'élément actif 3 est plus souple que l'élément support. Outre une moindre rigidité en raison du choix des matériaux le constituant, certaines configurations confèrent une plus grande souplesse à l'élément actif 3 afin de favoriser sa déformation contre l'élément support 4.

Ainsi, l'élément actif 3 peut être principalement constitué d'un matériau piézocomposite, composé de plots de céramique piézoélectrique dans une matrice polymère. De plus, l'élément actif 3 peut comporter des zones de souplesse en polymère élastique, par exemple en silicone. Ces zones de souplesses peuvent correspondre à des entailles réalisées dans l'élément actif 3 comblées par un polymère élastique. Les figures 4a à 4e représentent quelques motifs de zones de souplesse pour un élément actif 3 en forme de calotte sphérique. Ces zones de souplesse peuvent encore correspondre à la matrice polymère d'une structure piézocomposite, ou encore à des zones structurées mécaniquement, par exemple par réalisation d'entailles alternées sur les deux faces de l'élément actif 3 de sorte à lui donner plus d'élasticité.

Sur la figure 4a, des entailles radiales perpendiculaires 41 sont réalisées afin de diviser l'élément actif 3 en quart. Sur la figure 4b, des entailles radiales perpendiculaires 42 ne rejoignent pas le centre de l'élément actif 3. Sur la figure 4c, ces mêmes premières entailles 42 sont complétées par des secondes entailles 43, également radiales, alternée avec les premières entailles 42. Cependant, les secondes entailles 43 sont moins étendues en direction du centre de l'élément actif 3 afin de ne pas trop fragiliser le centre de l'élément actif 3. La figure 4d présente des entailles 44 concentriques centrées sur le centre de l'élément actif 3. La figure 4e présente une configuration dans laquelle des entailles radiales 45 rejoignent un évidement central au centre de l'élément actif 3 et sont terminées à leur extrémité distale par des sections d'entailles concentriques 46. On peut aussi définir un ensemble de transducteurs élémentaires rigides organisés en réseau dans une matrice de polymère souple.

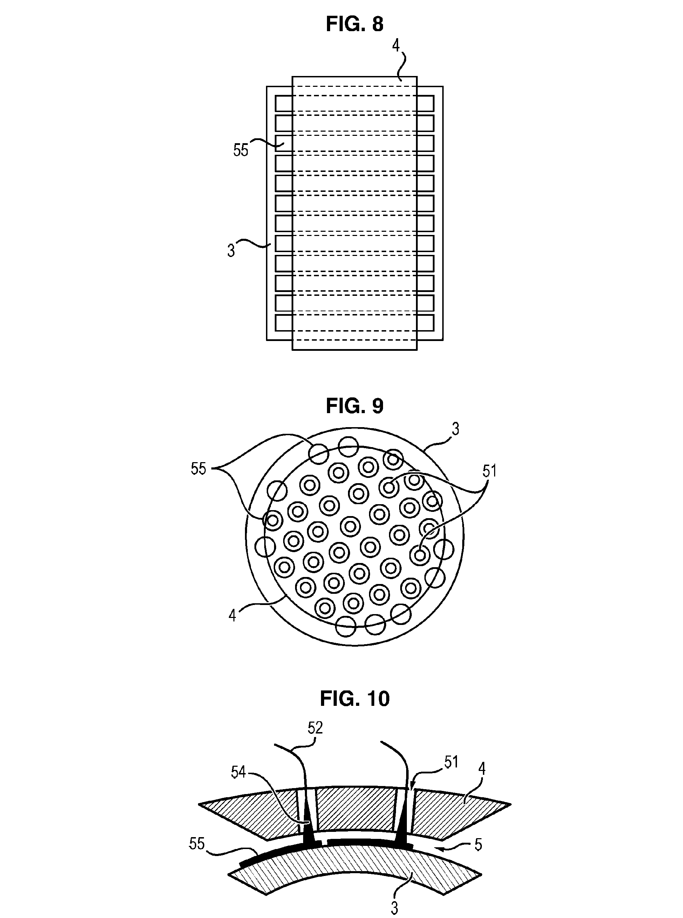

L'élément actif 3 présente une ou plusieurs électrode(s) sur sa face arrière afin de définir un ou plusieurs transducteur(s) élémentaire(s) correspondant(s). Lorsque la pluralité d'électrodes définit un réseau de transducteurs élémentaires selon une dimension ainsi qu'illustré sur la figure 8, la géométrie de l'élément support 4 peut alors être conçue pour assurer le contact dudit élément support 4 avec l'élément actif 3, tout en laissant accessible une partie au moins des électrodes 55, par exemple leur extrémités dépassant de l'élément support 4. Des contacts électriques peuvent alors être repris au niveau de ces extrémités d'électrodes 55.

Dans le cas où l'élément actif 3 présente un réseau de transducteurs élémentaires définis par des électrodes 55 prenant la forme d'un réseau selon deux dimensions, ainsi qu'illustré par la figure 9, l'élément support 4 peut alors recouvrir entièrement la surface de certaines électrodes 55. La reprise de contact électrique ne peut donc pas se faire directement par soudure de fils sur ces électrodes 55 recouvertes. L'aménagement de pistes électriques des électrodes 55 recouvertes vers la périphérie pose plusieurs problèmes, notamment de perte de surface active entre les électrodes pour faire passer les pistes, de risque d'inhomogénéité de comportement électrique en raison de des longueurs de pistes différentes entre électrodes, encombrement accru en diamètre...

Les figures 9 et 10 illustrent la reprise des contacts électriques au niveau de chaque électrode à travers des orifices aménagés dans l'élément support 4. Ainsi, l'élément actif 3 comporte une pluralité d'électrodes 55 sur sa face arrière, et l'élément support 4 comporte une pluralité d'orifices traversant 51 en regard d'au moins une partie de ladite pluralité d'électrodes, en l'occurrence les électrodes 55 recouvertes par l'élément support 4. Des fils de reprise 52 traversent ces orifices traversant 51 et sont chacun reliés au moyen d'une soudure 54 à une électrode 55 arrangée sur la face arrière de l'élément actif 3.

Or, l'élément actif 3 et l'élément support 4 sont en appui simple, donc non solidaires. Ces deux éléments sont amenés à bouger l'une par rapport à l'autre, même si les déplacements en question sont faibles, de l'ordre de quelques dixièmes de millimètres au maximum. Ces déplacements relatifs se produisent au moment de la mise en contact de l'élément actif 3 et de l'élément support 4 par précontrainte ou par dépression ou lors de la dilatation de la partie active en fonctionnement.

La liaison soudée étant fixe par rapport à l'élément actif 3, un volume suffisant doit être aménagé dans l'élément support 4 au-dessus de chaque électrode pour faire passer les fils 52, pour s'assurer que les points de soudure 54 en surépaisseur de l'élément actif 3 ne gênent pas la mise en contact de l'élément support 4 et de l'élément actif 3 et pour s'assurer que les points de soudure ne soient pas cisaillés par des déplacements relatifs de l'élément support 4 et de l'élément actif 3 l'un par rapport à l'autre.

Une autre option consiste à réaliser des reprises par contact, par exemple en utilisant des contacts à ressort fixés dans l'élément support 4, contacts qui ont une course suffisante entre l'élément actif 3 et l'élément support 4, et qui peuvent être translatés par rapport à l'élément actif 3 du transducteur.

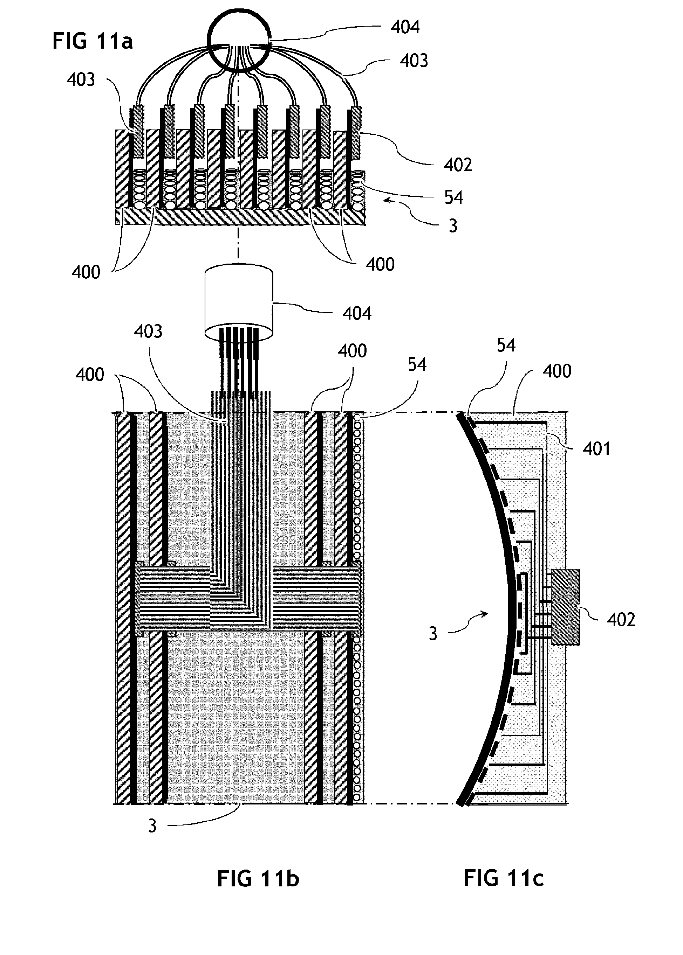

Les figures 11a, 11b et 11c représentent respectivement une vue de côté, de dessus et en coupe d'un transducteur selon un mode de réalisation possible de l'invention, dans laquelle l'élément support 4 est composé d'une pluralité de sous-éléments support 400. Ces sous-éléments support 400 sont mécaniquement reliés au boîtier, et chacun desdits sous-éléments support 400 est au contact de l'élément actif 3.

Plus précisément, chacun des sous-éléments support 400 présente sur une face avant conformée de sorte à ce que la face arrière complémentaire de l'élément actif 3 soit en appui simple sans couplage acoustique significatif avec la face avant dudit sous-élément support 400, de même que précédemment, et la face avant du sous-élément support 400 et la face arrière de l'élément actif 3 sont conformées pour définir entre elles, lorsque le sous-élément support 400 et l'élément actif 3 sont en appui simple, une couche discontinue de gaz 5 d'une épaisseur moyenne comprise entre 0,5 µm et 50 µm suffisamment faible pour favoriser un couplage thermique entre le sous-élément support 400 et l'élément actif 3.

De préférence, les sous-éléments support 400 sont agencés de sorte à maximiser la surface d'appui de l'élément support 4 qu'ils constituent avec l'élément actif 3. Ainsi, la surface de l'élément actif 3 sans support est minimisée. Par exemple, les sous-éléments support 400 sont placés ici parallèlement les uns par rapport aux autres, mais d'autres configurations peuvent être envisagées. De fait, plus la surface d'appui entre l'élément actif 3 et l'élément support 4 composé des sous-éléments 400 est importante, meilleur sera le renfort par l'élément support 4 et le couplage thermique.

Les sous-éléments support 400 peuvent présenter les mêmes caractéristiques, seuls ou par l'élément support 4 qu'ils constituent, qu'un élément support 4 précédemment décrit. Notamment, les sous-éléments support 400 peuvent être couplés au boîtier par contact direct ou via un éventuel drain thermique comme précédemment décrit. Il est à noter d'ailleurs que dans tous les cas, le boîtier peut lui-même être drainé thermiquement par un système de refroidissement.

Les sous-éléments support 400 sont ici fixés au boîtier afin de constituer l'élément support 4. Les sous-éléments support 400 sont ainsi mécaniquement reliés entre eux par le boîtier.

Chaque sous-élément support 400 se compose d'un élément de structure supportant des pistes 401 connectées d'une part par des soudures 54 aux électrodes des éléments piézo-électriques de l'élément actif 3 et d'autre part à un connecteur 402. Des circuits flexibles 403 assurent l'interconnexion entre les connecteurs 402 et un câble coaxiale 404.

L'élément actif 3 est ici un réseau matriciel d'éléments piézo-électriques. De préférence, il est prévu un sous-élément support 400 pour chaque colonne d'éléments piézo-électrique afin d'en assurer la reprise électrique au moyen des pistes 401, permettant d'allouer une ligne à chaque piste 401 du sous-élément support 400.

Une telle configuration permet de faciliter le montage du transducteur, qui peut notamment être réalisé par la mise en place successive de chaque sous-élément support 400 accompagnée de l'interconnexion électrique dudit sous-élément support 400 avec les éléments piézo-électriques qui lui sont affectés. En outre, cette configuration permet d'utiliser des solutions d'interconnexions électriques compactes.

De plus, la subdivision de l'élément support 4 en sous-éléments support 400 parallèles permet de s'adapter à l'aspect matriciel de l'élément actif 3.

L'invention concerne également un procédé d'utilisation d'un transducteur 1 selon l'invention, comportant
- une émission d'un faisceau d'ondes ultrasonores par l'élément actif 3, et
- un support de l'élément actif 3 par un élément support 4 sans couplage acoustique significatif, éventuellement accompagnée d'un drainage thermique par l'élément support 4.

## Revendications

1. Transducteur (1) ultrasonore comportant :
- un boîtier (2),
- un élément actif (3) pour générer et/ou recevoir des ondes ultrasonores, et
- un élément support (4) situé à l'arrière de l'élément actif (3), **caractérisé en ce que** l'élément support (4) comporte une face avant complémentaire d'une face arrière de l'élément actif (3), ledit élément support (4) étant conformé de sorte à ce que la face arrière complémentaire de l'élément actif (3) soit en appui simple sans couplage acoustique significatif avec la face avant dudit élément support (4), l'élément actif (3) et l'élément support (4) étant couplés thermiquement, et dans lequel la face avant de l'élément support (4) et la face arrière de l'élément actif (3) sont conformées pour définir entre elles, lorsque l'élément support (4) et l'élément actif (3) sont en appui simple, une couche discontinue de gaz (5) d'une épaisseur moyenne comprise entre 0,5 µm et 50 µm suffisamment faible pour favoriser un couplage thermique entre l'élément support (4) et l'élément actif (3).

2. Transducteur (1) selon la revendication précédente, dans lequel la couche discontinue de gaz (5) correspond au volume de gaz résiduel créé par des rugosités de la face avant de l'élément support (4) et de la face arrière de l'élément actif (3) lorsque l'élément support (4) et l'élément actif (3) sont en appui simple.

3. Transducteur (1) selon l'une des revendications 1 ou 2, comprenant en outre des organes de maintien en dépression adaptés pour maintenir la couche discontinue de gaz (5) à une pression inférieure d'au moins 200 mbar à la pression extérieure à laquelle est soumise la face avant de l'élément actif 3.

4. Transducteur (1) selon l'une quelconque des revendications précédentes, dans lequel l'écart moyen arithmétique des rugosités par rapport à la ligne moyenne d'au moins une face parmi la face arrière de l'élément actif (3) et la face avant de l'élément support (4) est compris entre 0,1 µm et 20 µm.

5. Transducteur (1) selon l'une quelconque des revendications précédentes, dans lequel l'élément actif (3) est encastré dans le boîtier (2) par l'intermédiaire d'au moins un joint élastique (6), et un effort mécanique de poussée de l'élément support (4) par rapport au boîtier (2) est généré au moyen d'éléments de plaquage (20, 30) afin de maintenir l'élément actif (3) en appui sur l'élément support 4 par une réaction dudit joint élastique (6) à l'effort mécanique.

6. Transducteur (1) selon l'une quelconque des revendications précédentes, dans lequel l'élément actif (3) comporte des zones de souplesse.

7. Transducteur (1) selon l'une quelconque des revendications précédentes, équipé en outre d'un système (11, 12, 13, 14, 15, 16) de refroidissement mettant en oeuvre la circulation d'un fluide caloporteur.

8. Transducteur (1) selon l'une quelconque des revendications précédentes, dans lequel l'élément actif (3) comporte une pluralité d'électrodes (55) sur sa face arrière, **caractérisé en ce que** l'élément support (4) comporte une pluralité d'orifices (51) traversant en regard d'au moins une partie de ladite pluralité d'électrodes (55).

9. Transducteur (1) selon l'une quelconque des revendications précédentes, dans lequel l'élément support (4) est composé de matériaux amagnétiques compatibles avec une utilisation en imagerie par résonnance magnétique.

10. Transducteur (1) selon l'une quelconque des revendications précédentes dans lequel l'élément support est composé d'une pluralité de sous-éléments support (400) mécaniquement reliés au boîtier, chacun desdits sous-éléments support (400) étant au contact de l'élément actif (3).

11. Procédé d'utilisation d'un transducteur (1) selon l'une quelconque des revendications précédentes, comportant :
• une émission d'un faisceau d'ondes ultrasonores par l'élément actif (3), et
• un support de l'élément actif (3) par un élément support (4) sans couplage acoustique significatif.
